(19) Europäisches Patentamt
European Patent Office
Office européen des brevets

(11) **EP 4 660 654 A1**

(12) **EUROPEAN PATENT APPLICATION**

(43) Date of publication:
**10.12.2025   Bulletin 2025/50**

(21) Application number: **25181167.5**

(22) Date of filing: **05.06.2025**

(51) International Patent Classification (IPC):
**G01R 33/30** (2006.01)    **A61B 5/00** (2006.01)
**G01R 33/36** (2006.01)

(52) Cooperative Patent Classification (CPC):
**G01R 33/3692; A61B 5/055; A61B 5/704;
G01R 33/307**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **07.06.2024   JP 2024092781**

(71) Applicant: FUJIFILM Corporation
Tokyo 106-8620 (JP)

(72) Inventors:
• ISHII, Koki
  Tokyo, 1068620 (JP)
• OTA, Atsushi
  Tokyo, 106862 (JP)
• CHUMAN, Takashi
  Tokyo, 1068620 (JP)

(74) Representative: **Dehns Germany Partnerschaft
mbB
Theresienstraße 6-8
80333 München (DE)**

(54) **PATIENT TABLE AND MAGNETIC RESONANCE IMAGING APPARATUS**

(57)     A patient table includes: a top plate (130) that moves with a subject (102) placed thereon; a leg portion (130B) that supports the top plate; a signal conversion unit (134) that is disposed inside the top plate and that is connected to a receive coil unit which receives a signal generated by the subject, the signal conversion unit including an A/D converter (160) that converts the signal obtained from the receive coil unit into a digital signal, and an electrical-to-optical converter (164) that converts the digital signal into an optical signal; an optical cable (136) that is disposed inside the top plate and the leg portion and that transmits the optical signal output from the signal conversion unit; and an optical wireless unit (46) that is disposed on the leg portion at a position outside a movement range of the top plate, is connected to the optical cable, and transmits the optical signal via optical wireless communication.

FIG. 7

## Description

## BACKGROUND OF THE INVENTION

1. Field of the Invention

The present disclosure relates to a patient table and a magnetic resonance imaging (MRI) apparatus.

2. Description of the Related Art

In an MRI apparatus, a subject placed in a static magnetic field is irradiated with high-frequency electromagnetic waves to excite nuclear spins inside the subject, for example, nuclear spins of hydrogen atoms, and nuclear magnetic resonance (NMR) signals generated in a case where the excited nuclear spins return to an equilibrium state are detected and subjected to signal processing, thereby visualizing a distribution of hydrogen atomic nuclei inside a living body as an image.

JP2014-61282A describes a magnetic resonance imaging apparatus comprising: a patient table on which a subject is placed; a gantry portion that supports a static magnetic field magnet and a gradient magnetic field coil; a receive coil unit that receives a magnetic resonance signal emitted from the subject; a conversion unit that converts the magnetic resonance signal output from the receive coil unit into a digital signal to generate magnetic resonance signal data; and a collection unit that collects the magnetic resonance signal data, in which the patient table or the gantry portion includes a coil port that connects the receive coil unit and the collection unit, and the conversion unit is provided on the coil port or a relay unit that relays between the receive coil unit and the coil port.

JP2014-230610A describes a magnetic resonance imaging apparatus including a patient table unit on which a subject is placed, the magnetic resonance imaging apparatus comprising: a digital processing unit that is disposed inside the patient table unit and that acquires an analog nuclear magnetic resonance signal from a receive coil unit, which detects a nuclear magnetic resonance signal emitted from the subject, and that digitizes the nuclear magnetic resonance signal; a first wireless communication unit that wirelessly transmits the nuclear magnetic resonance signal digitized by the digital processing unit; a second wireless communication unit that receives the nuclear magnetic resonance signal wirelessly transmitted by the first wireless communication unit; and an image reconstruction unit that reconstructs image data based on the nuclear magnetic resonance signal received by the second wireless communication unit.

JP2007-144192A describes a magnetic resonance imaging system comprising: a plurality of coil elements configured to supply each coil output signal based on a plurality of magnetic resonance response signals detected by the coil elements; and an optical link coupled to the plurality of coil elements in order to transmit at least one optical beam configured to transmit receive coil signal information through the air.

## SUMMARY OF THE INVENTION

Conventionally, weak magnetic resonance signals acquired by the receive coil unit have been transmitted via electrical cables and digitized at a position away from a gantry. Since analog transmission of signals raises concerns about noise interference and signal loss, a configuration has been proposed in which the signals are digitized inside a top plate close to the receive coil unit (JP2014-61282A and JP2014-230610A). In addition, since the MRI apparatus generates electromagnetic waves due to radio frequency (RF) irradiation in a strong magnetic field environment, using optical wireless communication with a frequency different from the RF irradiation frequency is effective for making the signal transmission, such as the nuclear magnetic resonance signals acquired by the receive coil unit, cable-free (JP2007-144192A). By converting the digitized signals into optical wireless signals inside the top plate, radio wave interference with the MRI apparatus can be avoided.

However, since the MRI apparatus moves the top plate to move the subject into a bore while performing imaging, it is necessary to dispose optical wireless transmitter and receiver at appropriate positions. That is, since the top plate operates in a state in which the subject is placed, it is necessary to consider where to dispose an optical wireless device, and there has been a problem in maintaining a stable optical link.

The present disclosure has been made in view of such circumstances, and an object of the present disclosure is to provide a patient table and a magnetic resonance imaging apparatus comprising an optical wireless unit that can suppress noise interference and signal loss and that can realize stable optical wireless communication.

According to a first aspect of the present disclosure, there is provided a patient table comprising: a top plate that moves with a subject placed thereon; a leg portion that supports the top plate; a signal conversion unit that is disposed inside the top plate and that is connected to a receive coil unit which receives a signal generated by the subject, the signal conversion unit including an A/D converter that converts the signal obtained from the receive coil unit into a digital signal, and an electrical-to-optical converter that converts the digital signal into an optical signal; an optical cable that is disposed inside the top plate and the leg portion and that transmits the optical signal output from the signal conversion unit; and an optical wireless unit that is disposed on the leg portion at a position outside a movement range of the top plate, that is connected to the optical cable, and that transmits the optical signal via optical wireless communication.

With the patient table according to the first aspect, the signal obtained from the receive coil unit is digitized by the signal conversion unit inside the top plate, converted into an optical signal, and transmitted, thereby enabling data transmission while suppressing noise interference and signal loss. Additionally, since the optical wireless unit is disposed at a position outside the movement range of the top plate, that is, at a position that is not shaded by the top plate, stable optical wireless communication is possible regardless of the position of the moving top plate.

According to a second aspect, in the patient table according to the first aspect, a configuration may be employed in which the optical wireless unit is disposed at an end portion of the leg portion in a direction in which the top plate moves.

According to a third aspect, in the patient table according to the first or second aspect, a configuration may be employed in which the optical wireless unit is disposed on a side surface portion of the leg portion.

According to a fourth aspect, in the patient table according to any one of the first to third aspects, a configuration may be employed in which the top plate includes a coil port including a connector to which a signal transmission cable of the receive coil unit is connected, and the signal conversion unit is disposed to correspond to the coil port.

According to a fifth aspect, in the patient table according to the fourth aspect, a configuration may be employed in which the top plate includes a plurality of the coil ports, and the signal conversion unit is provided for each of the plurality of coil ports.

According to a sixth aspect, in the patient table according to any one of the first to fifth aspects, a configuration may be employed in which a plurality of the signal conversion units and a plurality of the optical cables are provided.

According to a seventh aspect, in the patient table according to the sixth aspect, a configuration may be employed in which the optical wireless unit includes a switch for selecting a signal to be activated from the plurality of signal conversion units.

According to an eighth aspect, in the patient table according to the sixth or seventh aspect, a configuration may be employed in which the optical wireless unit includes a circuit that serializes signals obtained from a plurality of the receive coil units connected to the plurality of signal conversion units.

According to a ninth aspect, in the patient table according to any one of the first to eighth aspects, a configuration may be employed in which the signal conversion unit includes a digital processing circuit.

According to a tenth aspect, in the patient table according to the ninth aspect, a configuration may be employed in which the digital processing circuit includes a decimator that thins out digitized data.

According to an eleventh aspect, in the patient table according to the ninth or tenth aspect, a configuration may be employed in which the digital processing circuit includes a circuit that performs encoding.

According to a twelfth aspect, in the patient table according to any one of the first to eleventh aspects, a configuration may be employed in which a plurality of the optical wireless units are provided.

According to a thirteenth aspect, in the patient table according to any one of the first to twelfth aspects, a configuration may be employed in which the optical wireless unit includes an optical cable connector for wired connection to an optical communication apparatus disposed at a location away from the patient table.

According to a fourteenth aspect of the present disclosure, there is provided a magnetic resonance imaging apparatus comprising: the patient table according to any one of the first to thirteenth aspects; a gantry that generates a static magnetic field, a gradient magnetic field, and a high-frequency magnetic field in an imaging space; a second optical wireless unit that performs optical wireless communication with a first optical wireless unit which is the optical wireless unit; and an image reconstruction unit that reconstructs an image based on data of a nuclear magnetic resonance signal acquired via the second optical wireless unit.

With the magnetic resonance imaging apparatus according to the fourteenth aspect, the influence of noise components can be suppressed, thereby obtaining a high-quality image.

According to a fifteenth aspect, in the magnetic resonance imaging apparatus according to the fourteenth aspect, a configuration may be employed in which the second optical wireless unit is disposed on a ceiling or a wall of a room where the gantry is disposed.

According to a sixteenth aspect, in the magnetic resonance imaging apparatus according to the fifteenth aspect, a configuration may be employed in which the second optical wireless unit communicates with the first optical wireless unit via visible light communication and also serves as a light for the room.

According to a seventeenth aspect, in the magnetic resonance imaging apparatus according to any one of the fourteenth to sixteenth aspects, a configuration may be employed in which a plurality of the second optical wireless units are provided.

With the patient table of the present disclosure, noise interference and signal loss can be suppressed, thereby realizing stable optical wireless communication. In addition, with the magnetic resonance imaging apparatus of the present disclosure, the influence of noise components can be suppressed, thereby obtaining a high-quality image.

## BRIEF DESCRIPTION OF THE DRAWINGS

Fig. 1 is a perspective view showing an external appearance of an exemplary magnetic resonance imaging (MRI) apparatus.

Fig. 2 is a schematic diagram showing an internal configuration of the MRI apparatus.

Fig. 3 is an overview diagram showing a configuration example of a receive coil unit.

Fig. 4 is an explanatory diagram schematically showing a configuration of the MRI apparatus including a patient table according to an embodiment.

Fig. 5 is a plan view of the patient table.

Fig. 6 is a block diagram showing a configuration example of an A/D conversion unit.

Fig. 7 is an explanatory diagram of bidirectional optical wireless communication using an optical wireless unit.

Fig. 8 is a plan view showing a disposition example of the optical wireless unit.

Fig. 9 is a plan view showing an example of a case where a plurality of receive coil units are connected to coil ports.

Fig. 10 is a block diagram showing an example of the optical wireless unit that is connectable to the plurality of receive coil units.

Fig. 11 is an explanatory diagram showing an example of a patient table comprising a plurality of optical wireless units.

Fig. 12 is an explanatory diagram showing an example of an optical wireless unit comprising an optical cable connector.

## DESCRIPTION OF THE PREFERRED EMBODIMENTS

Preferred embodiments of the present invention will be described in detail below with reference to the accompanying drawings. In the following description and the accompanying drawings, components having the identical functional configuration will be denoted by the identical reference numerals, and repetitive descriptions will not be repeated.

Overview of Magnetic Resonance Imaging (MRI) Apparatus

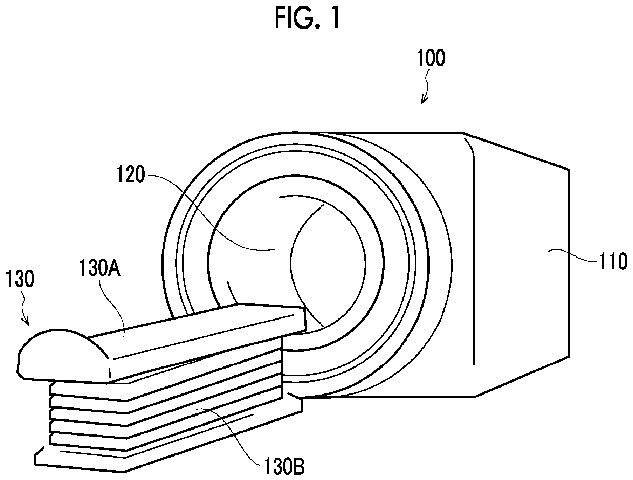

Fig. 1 is a perspective view showing an external appearance of an exemplary MRI apparatus 100. The MRI apparatus 100 comprises a gantry 110 which is an apparatus main body, and a patient table 130. The patient table 130 comprises a top plate 130A and a leg portion 130B that supports the top plate 130A, and is disposed in front of a bore 120, which is a cylindrical imaging space provided in the gantry 110. The top plate 130A can be moved into the bore 120 and moved out of the bore 120 by a top plate drive mechanism (not shown) provided in the leg portion 130B. The patient table 130 may be fixed to the gantry 110 or may be a movable patient table (dockable patient table) that is attachable to and detachable from the gantry 110.

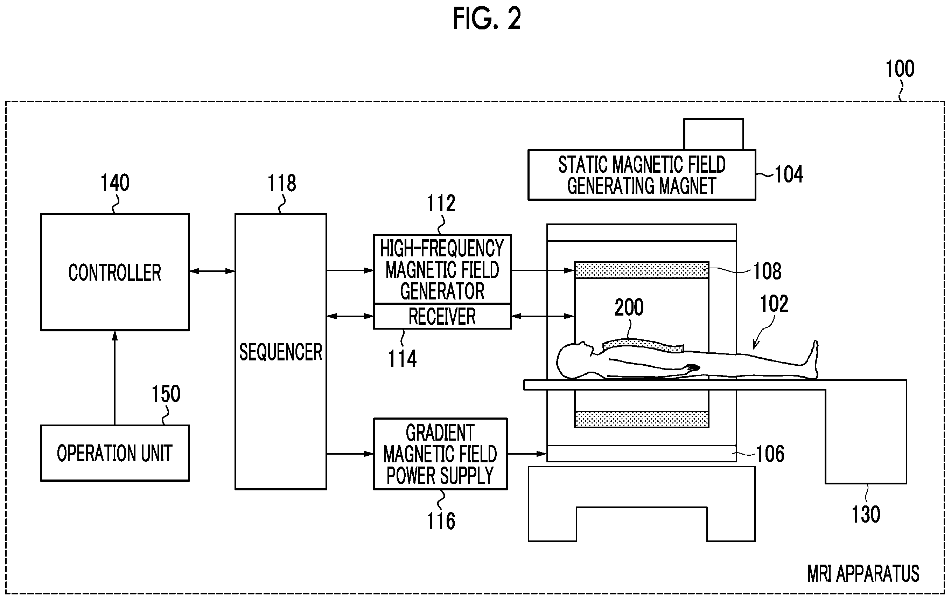

Fig. 2 is a diagram showing a schematic configuration of an inside of the MRI apparatus 100. The MRI apparatus 100 comprises a static magnetic field generating magnet 104, a gradient magnetic field coil 106, a radio frequency (RF) transmit coil 108, and a receive coil unit 200. A subject 102 is placed on the top plate 130A of the patient table 130 and is disposed in the imaging space. That is, by moving the top plate 130A on which the subject 102 is placed to the bore 120, an examination site of the subject 102 is moved to be located at the center of a static magnetic field in the bore 120.

The static magnetic field generating magnet 104 generates a uniform static magnetic field in the imaging space. The static magnetic field generating magnet 104 includes a static magnetic field generating source of a permanent magnet type, a normal conducting type, or a superconducting type. The gradient magnetic field coil 106 generates a gradient magnetic field in the imaging space. The gradient magnetic field coil 106 is composed of gradient magnetic field coils in three-axis directions of X, Y, and Z in a real space coordinate system (stationary coordinate system). Each gradient magnetic field coil is connected to a gradient magnetic field power supply 116 and is supplied with a current. As a result, the gradient magnetic field is generated in the three-axis directions of X, Y, and Z.

The RF transmit coil 108 is a coil that irradiates the subject 102 with a high-frequency magnetic field pulse (RF pulse). The RF transmit coil 108 is connected to a high-frequency magnetic field generator 112 and is supplied with a high-frequency pulse current. The high-frequency magnetic field generator 112 is driven in accordance with a command from a sequencer 118, modulates the amplitude of a high-frequency pulse, and supplies the amplified high-frequency pulse to the RF transmit coil 108.

The sequencer 118 transmits commands to the high-frequency magnetic field generator 112 and the gradient magnetic field power supply 116 in accordance with an imaging pulse sequence to generate the high-frequency magnetic field and the gradient magnetic field, respectively. The generated high-frequency magnetic field is applied to the subject 102 as a pulsed high-frequency magnetic field (RF pulse) through the RF transmit coil 108. As a result, nuclear magnetic resonance (NMR) phenomena are induced in the spins of atoms that constitute biological tissues of the subject 102.

The receive coil unit 200 is a coil that receives echo signals (referred to as NMR signals) emitted by the NMR phenomena of the spins of atoms that constitute the biological tissues of the subject 102. In Fig. 2, an example of a blanket-type receive coil unit 200 applied to imaging of a chest and an abdomen is shown; however, a form of the receive coil unit may be applied differently depending on the examination site. For example, receive coil units for imaging various sites, such as a head, a spine, an abdomen, a leg, and an arm, can be used. The number of receive coil units used for a single imaging operation may be one or plural, and a plurality of receive coil units for imaging different sites may be used at the same time. The receive coil unit may be simply called a receive coil. The NMR signal generated from the subject 102 is received by the receive coil unit 200 and is subjected to A/D conversion by a receiver 114.

The sequencer 118 controls each unit to operate at a pre-programmed timing and intensity. Among programs, a program that particularly describes the timing and intensity of RF pulses, gradient magnetic fields, and signal reception is called a pulse sequence. Various pulse sequences are known depending on the purpose, but a detailed description thereof will be omitted here.

A controller 140 controls the operation of the MRI apparatus 100 via the sequencer 118 and receives a signal from the receiver 114 to perform various types of signal processing, such as image reconstruction. The receiver 114 converts the signal obtained from the receive coil unit 200 into raw data and then transmits the raw data to the controller 140, and the raw data is also referred to as the echo signal or measurement data.

The controller 140 can be configured using a computer. The computer applied to the controller 140 may be a personal computer or a workstation.

The controller 140 accepts various instruction inputs from an operation unit 150 to perform overall control of the units of the MRI apparatus 100 and to perform processing of converting the echo signal in a spatial frequency domain received via the sequencer 118 into a real-space image through inverse Fourier transformation, and the like, thereby generating an MRI image.

The operation unit 150 includes a mouse, a keyboard, and the like and functions as a part of a graphical user interface (GUI) that accepts an input from an operator using a display operation window of a display (not shown). That is, the operation unit 150 and the display function as the GUI for the operator to start and stop (pause) the MRI apparatus 100, select pulse sequences, and input imaging conditions, processing conditions, and the like.

Overview of Receive Coil Unit

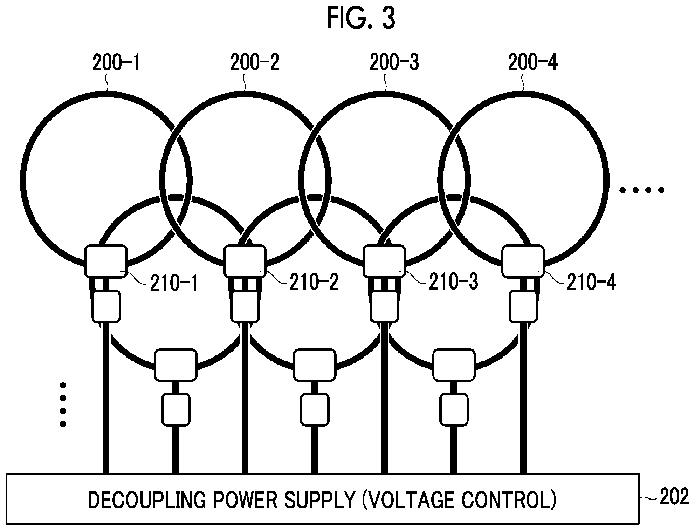

Fig. 3 is an overview diagram showing a configuration example of the receive coil unit 200. The receive coil unit 200 may be, for example, a flexible, thin, and lightweight coil unit capable of covering a wide imaging range of the chest and the abdomen of the subject 102. The receive coil unit 200 is a multi-channel array coil. In the receive coil unit 200, a plurality of loop-shaped coil elements 200-1, 200-2, 200-3, 200-4, ..., and 200-n that function as antennas that receive the NMR signals are disposed in a two-dimensional array, and decoupling circuits 210-1, 210-2, 210-3, 210-4, ..., and 210-n are provided to correspond to the respective coil elements 200-j (j = 1, 2, 3, 4, ..., and n).

Each of the plurality of coil elements 200-j functions as an antenna that receives the NMR signal generated from the biological tissue of the subject 102. Each coil element 200-j is adjusted to resonate at a specific frequency. The specific frequency is determined by the atomic nucleus (typically, a hydrogen atomic nucleus) to be observed in the biological tissue and the magnetic field intensity.

The shape, the number (the number of channels), and the disposition form of the coil element 200-j are not limited to the example shown in Fig. 3. In a case of a receive coil unit for the abdomen, the number of coil elements 200-j may range, for example, from 16 to 128.

The plurality of decoupling circuits 210-j (j = 1, 2, 3, 4, ..., and n) include voltage-driven field effect transistors (FETs), and the FET in each decoupling circuit is controlled ON/OFF by a drive voltage supplied from a decoupling power supply 202.

Description of Configuration of MRI Apparatus Including Patient Table According to Embodiment

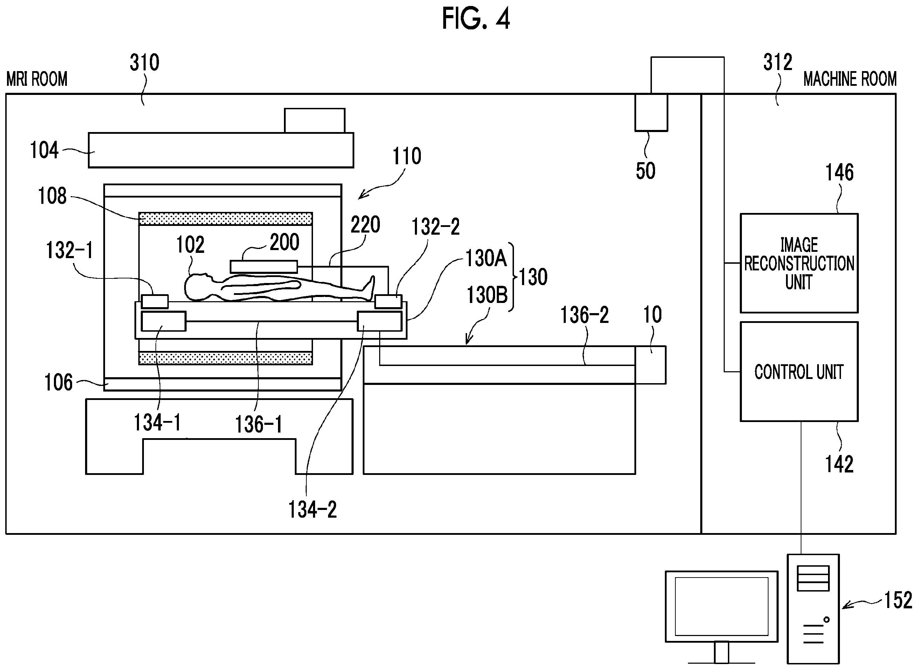

Fig. 4 is an explanatory diagram schematically showing a configuration of the MRI apparatus 100 including the patient table 130 according to the embodiment. The gantry 110 and the patient table 130 are disposed inside an MRI room 310, which is an examination room for MRI examinations. The MRI room 310 is an example of a "room where the gantry is disposed".

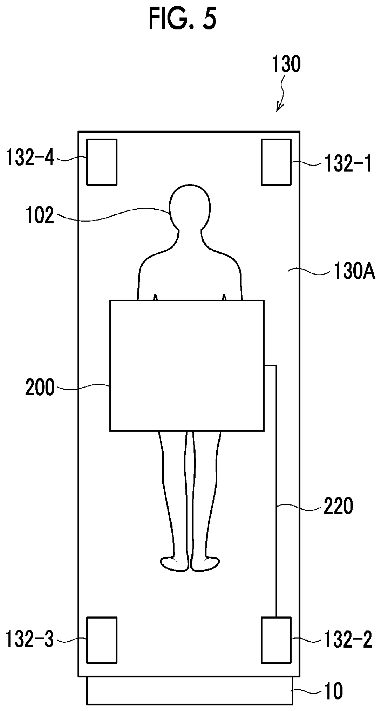

Fig. 5 is a plan view of the patient table 130 and shows a state before the top plate 130A is moved into the bore 120 (a state of an initial position of the top plate 130A). The upper direction of Fig. 5 is a direction of the gantry 110.

Coil ports 132-1, 132-2, 132-3, and 132-4 are disposed on the top plate 130A. A coil port 132-k (k = 1, 2, 3, and 4) is a connector unit including a connector to which a signal transmission cable 220 of the receive coil unit 200 is connectable. In Fig. 5, an example is shown in which the coil ports 132-k are disposed at four corners of the top plate 130A, but the number and the disposition location of the coil ports are not limited to the example of Fig. 5. The coil ports are disposed at one or more locations, preferably at a plurality of locations, on the top plate 130A.

The signal transmission cable 220 is an electrical cable that transmits the signal of each channel output from the receive coil unit 200. The signal transmission cable 220 may be connected to the receive coil unit 200 via a connector or may be integrated with the receive coil unit 200.

The signal transmission cable 220 of the receive coil unit 200 is connected to any coil port 132-k on the top plate 130A. In Fig. 5, an example is shown in which the signal transmission cable 220 is connected to the coil port 132-2 at the lower right, but the signal transmission cable 220 may be connected to another coil port 132-1, 132-3, or 132-4.

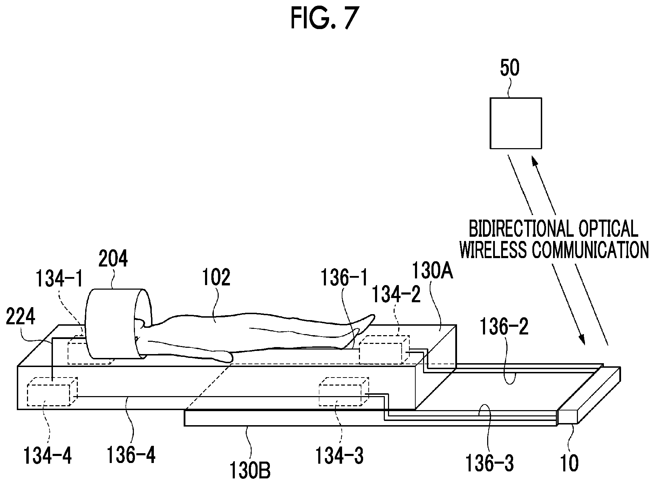

An A/D conversion unit 134-k corresponding to each of the coil ports 132-k (refer to Figs. 4 and 7) is provided inside the top plate 130A. The A/D conversion unit 134-k may be disposed directly below the corresponding coil port 132-k. The A/D conversion unit 134-k may be incorporated into the coil port 132-k.

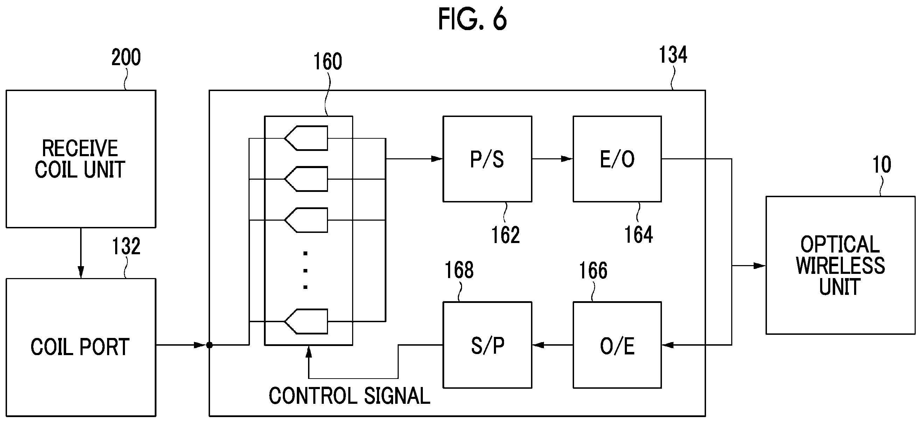

A specific configuration example of the A/D conversion unit 134-k will be described below with reference to Fig. 6. Each A/D conversion unit 134-k comprises an electrical-to-optical (E/O) converter 164 and an optical-to-electrical (O/E) converter 166. In addition, each A/D conversion unit 134-k is a signal conversion module comprising a parallel-to-serial (P/S) converter 162 and a serial-to-parallel (S/P) converter 168.

An optical signal output from the A/D conversion unit 134-k is transmitted to an optical wireless unit 10 using an optical cable 136-k. The term "optical cable" is synonymous with an optical fiber cable.

The optical wireless unit 10 is an optical communication module that transmits and receives an optical signal via optical wireless communication, is disposed on the patient table 130, and performs bidirectional optical wireless communication with an optical wireless unit 50.

The optical wireless unit 10 is disposed at a position on the patient table 130 where communication is not interrupted by shading due to the gantry 110 or the moving top plate 130A, from the viewpoint of communication stability. Specifically, the optical wireless unit 10 is disposed at an end portion of the leg portion 130B on a side opposite to the gantry 110 or on a side surface portion of the leg portion 130B, which is outside a movement range of the top plate 130A in plan view. The movement range of the top plate 130A is a region that may be covered by the moving top plate 130A, and can be understood as a movable region of the top plate 130A. By disposing the optical wireless unit 10 at a position that protrudes outside the movement range of the top plate 130A in plan view, communication stability with the optical wireless unit 50 can be enhanced. The leg portion 130B is a portion that does not move in a state of being connected to the gantry 110. The term "end portion" includes an end and surrounding parts. Figs. 4 and 5 show an example in which the optical wireless unit 10 is disposed at an end (rear end) of the leg portion 130B on the side opposite to the gantry 110.

The optical cable 136-k is disposed inside the top plate 130A and the leg portion 130B and is extended from the A/D conversion unit 134-k to the optical wireless unit 10 at the end of the leg portion 130B.

The optical wireless unit 10 is connected to a plurality of A/D conversion units 134-k via the optical cables 136-k (refer to Fig. 7). In this case, the optical wireless unit 10 comprises a switch for selecting a signal to be activated from the plurality of A/D conversion units 134-k.

The optical wireless unit 50 that forms an optical link with the optical wireless unit 10 is disposed on a ceiling of the MRI room 310 or a wall surface of the MRI room 310. The optical wireless unit 50 is an optical communication module that transmits and receives an optical signal via optical wireless communication, similar to the optical wireless unit 10, and functions as an optical controller. A control unit 142 and an image reconstruction unit 146 of the MRI apparatus 100 are disposed in a machine room 312 and are connected to the optical wireless unit 50. The optical wireless unit 10 is an example of a "first optical wireless unit", and the optical wireless unit 50 is an example of a "second optical wireless unit".

The control unit 142 includes a processor that generates a control signal and a multiplexer. The processor may be configured using, for example, a field programmable gate array (FPGA). The processor is not limited to the FPGA and may be configured using another programmable integrated circuit (IC) or digital signal processor (DSP), a combination thereof, or the like. The control unit 142 is connected to a console 152. The console 152 may include a computer that functions as the controller 140 and the operation unit 150 shown in Fig. 2.

Configuration Example of A/D Conversion Unit

Since the configurations of the A/D conversion units 134-k are common, descriptions will be provided using an A/D conversion unit 134 in Fig. 6. Additionally, since the configurations of the coil ports 132-k are also common, descriptions will be provided using a coil port 132 in Fig. 6. A receive coil unit 204 or other types of receive coil units can be connected to the coil port 132, not just the receive coil unit 200.

Fig. 6 is a block diagram showing a configuration example of the A/D conversion unit 134. The A/D conversion unit 134 includes an A/D converter 160, the P/S converter 162, the E/O converter 164, the O/E converter 166, and the S/P converter 168. The A/D conversion unit 134 is an example of a "signal conversion unit". The E/O converter 164 is an example of an "electrical-to-optical converter".

The A/D converter 160 includes a plurality of A/D converters corresponding to the number of channels of the receive coil unit 200. The A/D converter 160 converts the analog signal received from the receive coil unit 200 through the coil port 132 into digital signal data. The A/D conversion unit 134 may comprise a decimator (not shown) that thins out the digital signal data converted by the A/D converter 160. The P/S converter 162 performs serialization (P/S conversion) for optical transmission. The P/S converter 162 may include a multiplexer and an encoding function. The E/O converter 164 converts the serialized signal data into an optical signal.

The optical signal output from the E/O converter 164 is transmitted to the optical wireless unit 10, is converted into an optical wireless signal by the optical wireless unit 10, and is transmitted to the optical wireless unit 50.

In addition, the optical wireless unit 10 receives the optical wireless signal transmitted from the optical wireless unit 50 and transmits the received signal to the A/D conversion unit 134. The signal received by the optical wireless unit 10 from the optical wireless unit 50 includes, for example, control signals for the A/D converter 160 and the like.

The optical signal transmitted from the optical wireless unit 10 to the A/D conversion unit 134 is converted into an electrical signal by the O/E converter 166. The S/P converter 168 performs deserialization (S/P conversion) of the O/E converted signal. The control signal for each channel output from the S/P converter 168 is transmitted to each control destination, and the operations of the A/D converter 160, the receive coil unit 200, and the like are controlled.

Overview of Bidirectional Optical Wireless Communication

Fig. 7 is an explanatory diagram of bidirectional optical wireless communication using the optical wireless units 10 and 50. In Fig. 7, an example is shown in which the receive coil unit 204 for the head is used instead of the receive coil unit 200. In Fig. 7, the coil port 132-k is not shown. The receive coil unit 204 is connected to the A/D conversion unit 134-4 via a signal transmission cable 224. The signal obtained from the receive coil unit 204 is digitized by the A/D conversion unit 134-4 and converted into an optical signal. The optical signal output from the A/D conversion unit 134-4 is transmitted to the optical wireless unit 10 via the optical cable 136-4, is modulated into an optical wireless signal by the optical wireless unit 10, and is transmitted from the optical wireless unit 10 via optical wireless communication.

The optical wireless unit 50 receives the optical wireless signal transmitted from the optical wireless unit 10, converts the received signal into an electrical signal, and transmits the electrical signal to the image reconstruction unit 146. Additionally, the optical wireless unit 50 converts the control signal output from the control unit 142 into an optical signal and transmits the optical signal via optical wireless communication.

The optical signal transmitted from the optical wireless unit 50 is received by the optical wireless unit 10, and the signal is transmitted from the optical wireless unit 10 to the A/D conversion unit 134-4 via the optical cable 136-4. The operation of the A/D conversion unit 134-4 is controlled by a control signal received via the optical wireless unit 10. In addition, the operation of the receive coil unit 204 is controlled by a control signal received via the optical wireless unit 10.

The same applies to the other A/D conversion units 134-1, 134-2, and 134-3, in a case where the receive coil unit is connected to the A/D conversion units 134-1, 134-2, and 134-3, which transmit and receive the optical signals to and from the optical wireless unit 10 via the optical cables 136-1, 136-2, and 136-3, respectively.

Overview of Operation of MRI Apparatus 100

An overview of the operation of the MRI apparatus 100 using the optical wireless units 10 and 50 (steps 1 to 11) is as follows. Here, a case where the patient table 130 is a movable dockable patient table is exemplified.

[Step 1] The patient table 130 is docked with the gantry 110.

[Step 2] A link (optical wireless communication connection) is established between the optical wireless units 10 and 50. The optical wireless communication technology applied may be, for example, light fidelity (LiFi). LiFi is a technology that realizes wireless bidirectional communication by modulating light at a high speed. LiFi can also support optical communication using not only visible light but also near-infrared light and can avoid the influence of electromagnetic interference as compared with wireless communication using radio waves, thereby enabling high-speed and stable communication.

[Step 3] The subject 102 is placed on the top plate 130A and moved to the imaging region.

[Step 4] A control signal is output from the control unit 142 based on the selected receive coil unit and the parameters input via the console 152.

[Step 5] The control signal output from the control unit 142 is optically wirelessly transmitted to the optical wireless unit 10 via the optical wireless unit 50.

[Step 6] The optical wireless unit 10 that has received the control signal transmits the control signal to the A/D conversion unit 134-k to which the receive coil unit 200 to be activated is connected. Here, as an example, a case where the control signal is transmitted to the A/D conversion unit 134-2 to which the receive coil unit 200 to be activated is connected will be described.

[Step 7] By executing the imaging pulse sequence, the biological signals (NMR signals) of the plurality of channels acquired by the receive coil unit 200 are input to the A/D conversion unit 134-2 inside the top plate 130A via the existing signal transmission cable 220, a connector (not shown), and the coil port 132-2.

[Step 8] The A/D converter 160, the P/S converter 162, and the E/O converter 164 in the A/D conversion unit 134-2 are operated by the control signal received from the optical wireless unit 10 to perform the A/D conversion, the P/S conversion, and the E/O conversion of each signal, and then the signal is transmitted to the optical wireless unit 10 via the optical cable 136-2 as an optical signal.

[Step 9] In the optical wireless unit 10, the activated signal received from the A/D conversion unit 134-2 to which the receive coil unit 200 is connected is converted into an optical wireless signal and transmitted to the optical wireless unit 50.

[Step 10] The optical wireless unit 50 performs O/E conversion on received data, and the data is transmitted from the optical wireless unit 50 to the image reconstruction unit 146.

[Step 11] The image reconstruction unit 146 performs processing of reconstructing an image from the received data and provides the image to the console 152. In this way, an MRI image, which is the reconstructed image, is displayed on a display device of the console 152.

Example of Control Signal Received by A/D Conversion Unit 134 from Optical Wireless Unit 10

For example, signals listed in [1] to [5] below are serialized and transmitted from the optical wireless unit 50 to the optical wireless unit 10, and the signals are restored (S/P conversion) by the S/P converter 168 in the A/D conversion unit 134 and then transmitted to each control destination.

[1] A sampling clock for the A/D converter 160 synchronized with an RF irradiation signal
[2] A control signal for the operating setting of the A/D converter 160
[3] A control signal for the setting of digital signal processing including decimation and encoding
[4] A control signal for the gain setting of a variable gain amplifier (VGA)
[5] A decoupler signal of the receive coil unit 200

Although not shown, the A/D conversion unit 134 comprises a VGA and a buffer. The control signal for the gain setting of the VGA is distributed to each VGA via the buffer after being restored by the S/P converter 168. Additionally, the decoupler signal of the receive coil unit 200 is restored by the S/P converter 168 and then distributed by a fan-out buffer, thereby performing decoupling control of each coil element 200-j.

Regarding Disposition Location of Optical Wireless Unit 10

In Figs. 4 and 5, an example has been described in which the optical wireless unit 10 is disposed at the rear end of the leg portion 130B (the end on the side opposite to the gantry 110), but the shape, the disposition location, and the number of the optical wireless unit 10 are not limited to the examples shown in Figs. 4 and 5.

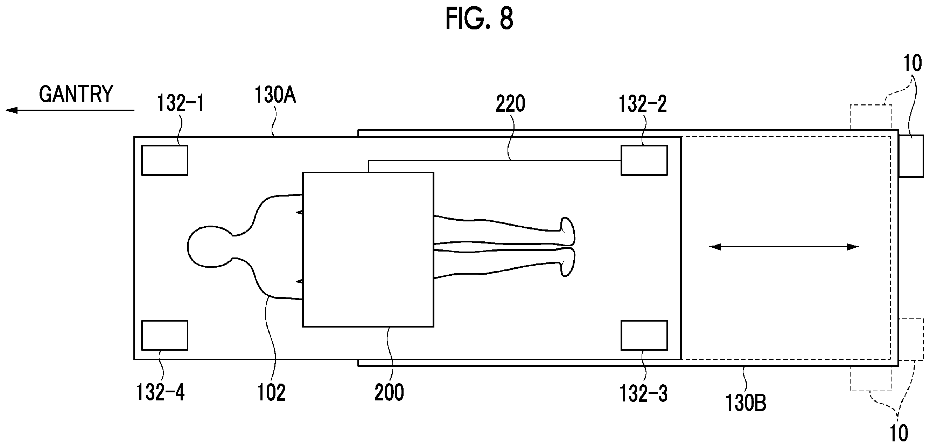

Fig. 8 is a plan view showing a disposition example of the optical wireless unit 10. The optical wireless unit 10 is disposed at a position that is not shaded by the moving top plate 130A. The "position that is not shaded" is a position that does not overlap with the movement range of the top plate 130A in plan view, that is, a position outside the movement range of the top plate 130A. The optical wireless unit 10 is disposed at a position that protrudes beyond the region of the top plate 130A in plan view in an initial position state of the top plate 130A before the top plate 130A is moved, and the disposition position is a position that protrudes beyond the region of the movement range of the top plate 130A in plan view even in a case where the top plate 130A is moved in the direction of the gantry 110. As an example of the disposition location satisfying such conditions, as shown in Fig. 8, the optical wireless unit 10 is disposed at the end portion of the leg portion 130B on the side opposite to the gantry 110.
In a case where a long side direction (longitudinal direction) of the top plate 130A, which is substantially rectangular in plan view, is defined as a Z-axis direction and a short side direction (lateral direction) is defined as an X-axis direction, and in a case where a gantry 110 side of both ends of the patient table 130 in the Z-axis direction is defined as a front end and the side opposite to the gantry 110 (a side away from the gantry 110) is defined as a rear end, the optical wireless unit 10 may be disposed at the rear end of the leg portion 130B that supports the top plate 130A or at the side surface portion close to the rear end, or at a plurality of locations thereof. Here, the "side surface portion" refers to the part of the side surface corresponding to the long side orthogonal to the X-axis direction. The bidirectional arrows parallel to the Z-axis direction shown in Fig. 8 indicate the movement of the top plate 130A.

Example of Plurality of Receive Coil Units Connected to Coil Ports

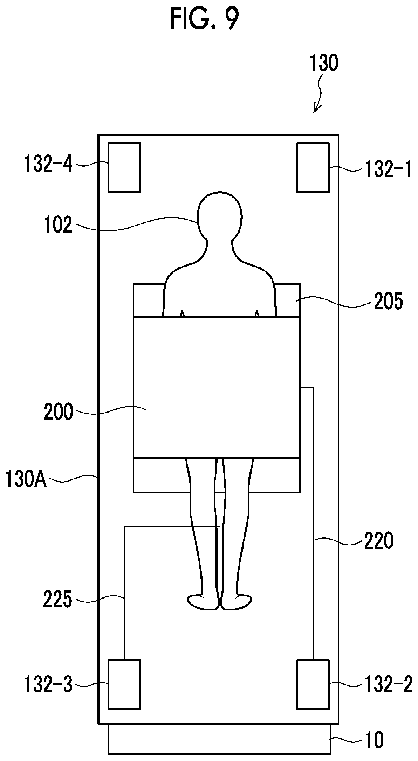

In the MRI apparatus 100, a plurality of receive coil units may be used for the subject 102. Fig. 9 is a plan view showing an example of a case where a plurality of receive coil units 200 and 205 are connected to the coil ports 132-2 and 132-3. In Fig. 9, the receive coil unit 200 for the abdomen and the receive coil unit 205 for the spine are illustrated, but the present disclosure is not limited to the combination thereof. In addition, three or more receive coil units may be used.
For the configuration shown in Fig. 9, elements common to those in Fig. 5 are denoted by the identical reference numerals, and repetitive descriptions will not be repeated. A signal transmission cable 225 of the receive coil unit 205 is connected to, for example, the coil port 132-3.
In a case where the plurality of receive coil units 200 and 205 are connected to the coil ports 132-2 and 132-3, a plurality of activated signals are selected by the optical wireless unit 10, and the signals obtained from each of the receive coil units 200 and 205 are serialized (or multiplexed) and transmitted via optical wireless communication.

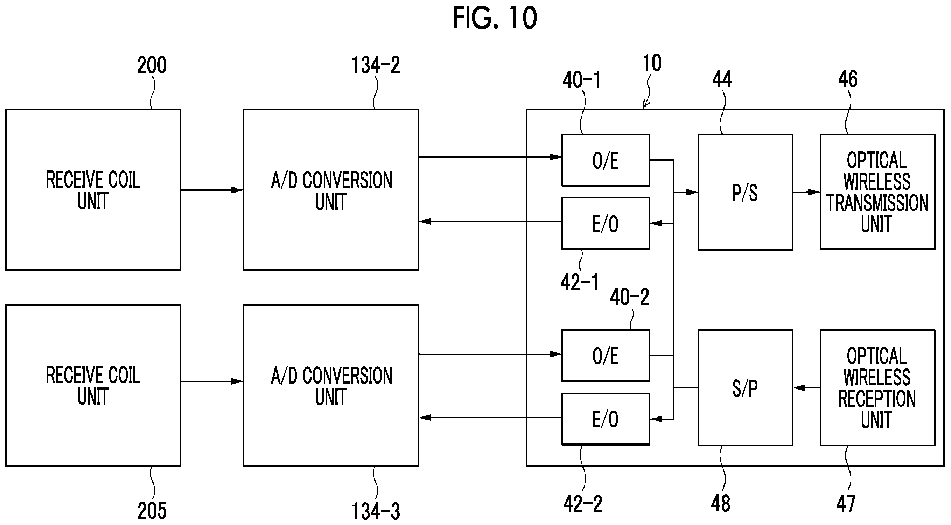

Fig. 10 is a block diagram showing an example of the optical wireless unit 10 that is connectable to the plurality of receive coil units 200 and 205. The optical wireless unit 10 comprises, for example, a plurality of O/E converters 40-1 and 40-2, a plurality of E/O converters 42-1 and 42-2, a P/S converter 44, an optical wireless transmission unit 46, an optical wireless reception unit 47, and an S/P converter 48. The optical wireless transmission unit 46 includes a light source, such as a light-emitting diode, and an optical modulator. The optical wireless reception unit 47 includes an optical sensor, such as a photodiode, and an amplification circuit. In Fig. 10, a configuration is shown in which two O/E converters 40-1 and 40-2 and two E/O converters 42-1 and 42-2 are provided, but a configuration may be employed in which two or more O/E converters and two or more E/O converters are provided in accordance with the number of connectable receive coil units.

An overview of the operation (steps 201 to 211) in a case where the plurality of receive coil units 200 and 205 are connected to the coil ports 132-2 and 132-3 is as follows.

[Step 201] First, the plurality of receive coil units 200 and 205 are connected to the plurality of coil ports 132-2 and 132-3 on the top plate 130A. As a result, the plurality of receive coil units 200 and 205 are connected to the corresponding A/D conversion units 134-2 and 134-3, respectively.

[Step 202] Next, the receive coil units 200 and 205 to be activated are selected by the console 152.

[Step 203] The control signal is output from the control unit 142 to each of the plurality of activated receive coil units 200 and 205 in accordance with the selection from the console 152.

[Step 204] The control signal output from the control unit 142 is optically wirelessly transmitted to the optical wireless unit 10 via the optical wireless unit 50.

[Step 205] The optical wireless unit 10 receives the control signal transmitted via optical wireless communication from the optical wireless unit 50 using the optical wireless reception unit 47 and performs deserialization of the received control signal using the S/P converter 48. The S/P converter 48 distributes the control signal for each of the receive coil units 200 and 205 and transmits the control signals to the E/O converters 42-1 and 42-2.

[Step 206] The control signals for the receive coil units 200 and 205 are subjected to E/O conversion by the E/O converters 42-1 and 42-2 and are transmitted to the A/D conversion units 134-2 and 134-3 via the optical cables 136-2 and 136-3, respectively. In this way, the control signals are transmitted to all of the plurality of A/D conversion units 134-2 and 134-3 to which the activated receive coil units 200 and 205 are connected.

[Step 207] The A/D conversion units 134-2 and 134-3 perform O/E conversion on the respective received control signals, operate devices such as the A/D converter 160 in accordance with the control signals, and digitize the biological signals obtained from the corresponding receive coil units 200 and 205. That is, similar to the above-mentioned steps 7 and 8, the A/D converters 160, the P/S converters 162, and the E/O converters 164 in the A/D conversion units 134-2 and 134-4 are operated by the control signals received from the optical wireless unit 10 to perform the A/D conversion, the P/S conversion, and the E/O conversion of the signal of each channel. In this way, the signal data obtained from the plurality of receive coil units 200 and 205 is converted into optical signals by the A/D conversion units 134-2 and 134-3 and is transmitted to the optical wireless unit 10 via the optical cables 136-2 and 136-3.

[Step 208] The optical wireless unit 10 receives the optical signals transmitted from the plurality of A/D conversion units 134-2 and 134-3 and performs P/S conversion by the P/S converter 44 after O/E conversion by the O/E converters 40-1 and 40-2.

[Step 209] The optical wireless transmission unit 46 of the optical wireless unit 10 converts the P/S-converted data into optical wireless data and transmits the optical wireless data to the optical wireless unit 50.

[Step 210] The optical wireless unit 50 performs O/E conversion and further performs S/P conversion on the received data, and then the data is transmitted from the optical wireless unit 50 to the image reconstruction unit 146 in the same manner as in step 10.

[Step 211] The image reconstruction unit 146 performs processing of reconstructing an image from the received data and provides the image to the console 152.

Regarding Configuration in Which Optical Wireless Unit 50 Also Serves as Light in MRI Room 310

The optical wireless unit 50 may also serve as a lighting fixture (light). For example, by employing a configuration in which a plurality of optical wireless units 50 are disposed on the ceiling of the MRI room 310 and each of the optical wireless units 50 performs visible light communication with the optical wireless unit 10, the optical wireless unit 50 can be used as a light for illumination in the MRI room 310.

In a case where there is an illumination device or another illumination equipment in the gantry 110 in addition to the optical wireless unit 50, the optical signals for visible light communication can be filtered based on a communication speed, thereby enabling communication without interference between light sources.

Regarding Configuration in Which Digital Processing Function Is Implemented in A/D Conversion Unit 134

The A/D conversion unit 134 may have a digital processing function and may transmit a signal in a communication format suitable for optical wireless communication. The A/D conversion unit 134 comprises, for example, a digital processing circuit that performs processing such as decimation and encoding. The encoding may be, for example, conversion from 8 bits to 10 bits (8b/10b conversion). The A/D conversion unit 134 comprising the digital processing circuit digitizes the analog signal acquired from the receive coil unit using the A/D converter 160 and then thins out the data through decimation. Then, the thinned-out data is encoded (such as 8b/10b conversion) to prevent the occurrence of continuous bits. Through such digital processing, the signal can be adjusted to correspond to a transmission amount and a transmission speed of the optical wireless communication. Specific processing examples will be described below.

For example, in a case where data of 16 bits, 100 Msps (samples per second), and 16 channels is transmitted, in order to achieve an optical wireless signal of 10 Gbps or less, the following equation applies:

$$16 \times 100M \times (1/4) \times (10/8) \times 16 = 8.0 \text{ Gbps.}$$

As shown in the equation, by setting the decimation factor to 4 and encoding to 8b/10b, the specifications can be satisfied.

Example 1 of Patient Table Comprising Plurality of Optical Wireless Units

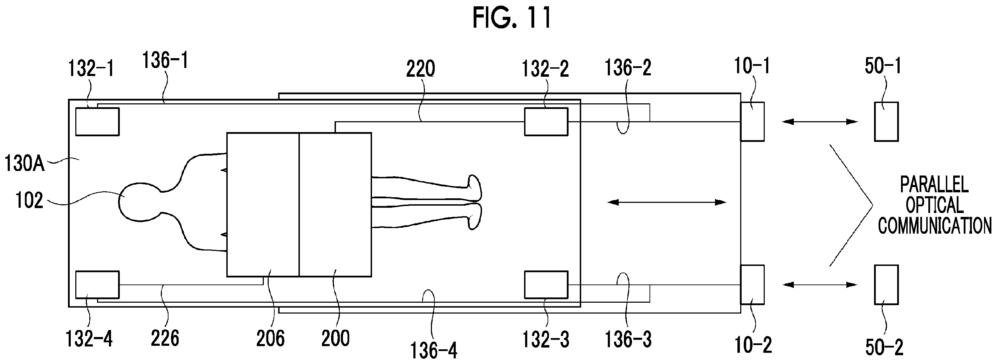

Fig. 11 is an explanatory diagram showing an example of a patient table 130 comprising a plurality of optical wireless units 10-1 and 10-2. In the patient table 130 shown in Fig. 11, the plurality of optical wireless units 10-1 and 10-2 are disposed at the rear end of the leg portion 130B. The optical wireless unit 10-1 is connected to the A/D conversion units 134-1 and 134-2 (not shown in Fig. 11, refer to Fig. 7) corresponding to the coil ports 132-1 and 132-2 via the optical cables 136-1 and 136-2.

In addition, the optical wireless unit 10-2 is connected to the A/D conversion units 134-3 and 134-4 (not shown in Fig. 11, refer to Fig. 7) corresponding to the coil ports 132-3 and 132-4 via the optical cables 136-3 and 136-4.

That is, the signals digitized by the A/D conversion units 134-1 and 134-2 are transmitted to the optical wireless unit 10-1, and the signals digitized by the A/D conversion units 134-3 and 134-4 are transmitted to the other optical wireless unit 10-2. The functions of the optical wireless units 10-1 and 10-2 may be the same as the function of the optical wireless unit 10 shown in Fig. 10.

In a case of the example shown in Fig. 11, the signal obtained from the receive coil unit 200 is digitized by the A/D conversion unit 134-2 and is transmitted to the optical wireless unit 10-1. Additionally, the signal obtained from a receive coil unit 206 is digitized by the A/D conversion unit 134-4 and is transmitted to the optical wireless unit 10-2. In this way, large-volume data obtained from the plurality of receive coil units 200 and 206 is transmitted in parallel to fit within the optical wireless data transfer rate.

In addition, a plurality of optical wireless units 50-1 and 50-2 are disposed on the ceiling or the like of the MRI room 310, and similar to the optical wireless unit 50 shown in Fig. 4, each of the optical wireless units 50-1 and 50-2 is connected to the image reconstruction unit 146 and the control unit 142.

The optical wireless connection (optical link) is established between the optical wireless unit 50-1 and the optical wireless unit 10-1, and the optical wireless connection is established between the optical wireless unit 50-2 and the optical wireless unit 10-2, thereby enabling parallel optical communication through each optical link.

Example 2 of Patient Table Comprising Plurality of Optical Wireless Units

In Fig. 11, an example has been described in which the plurality of optical wireless units 10-1 and 10-2 are disposed at the rear end of the leg portion 130B, but a disposition form of the plurality of optical wireless units provided on the patient table 130 is not limited to the example in Fig. 11. For example, optical wireless units may be disposed on both sides of the end of the patient table 130 in the longitudinal direction, and the optical wireless unit to be used may be determined according to the direction in which the top plate 130A moves. In this case, for example, a configuration may be employed in which the same optical wireless unit 10 as that in Fig. 5 is disposed at the rear end of the leg portion 130B and another optical wireless unit is disposed at a front end portion of the top plate 130A.

Example of Optical Wireless Unit Comprising Optical Cable Connector

The optical wireless unit 10 disposed on the patient table 130 may also have an optical communication transmission and reception function via wired connection using an optical cable, in addition to the optical wireless transmission and reception function.

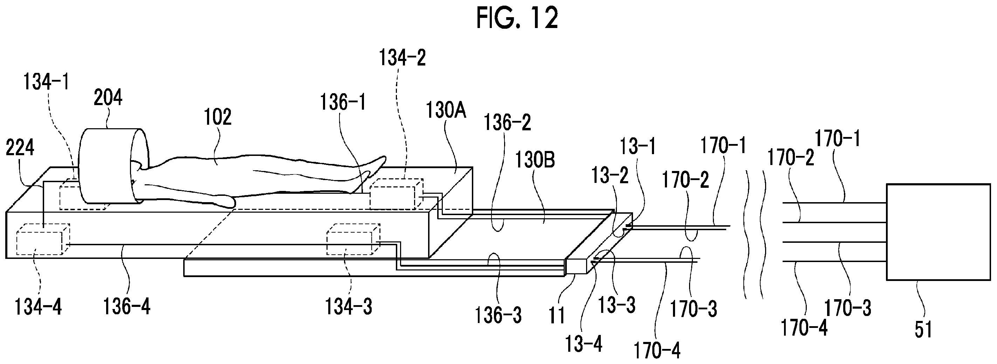

Fig. 12 is an explanatory diagram showing an example of an optical wireless unit 11 comprising optical cable connectors 13-1, 13-2, 13-3, and 13-4. The optical wireless unit 11 shown in Fig. 12 may be applied instead of the optical wireless unit 10 shown in Figs. 4 to 7. Differences from Fig. 7 will be described with reference to Fig. 12.

The optical wireless unit 11 comprises the optical cable connectors 13-1, 13-2, 13-3, and 13-4 for connection to a distant master apparatus 51, in addition to the same optical wireless communication function as that of the optical wireless unit 10.

An optical cable 170-k can be connected to the optical cable connector 13-k (k = 1, 2, 3, and 4), and the optical wireless unit 11 can be connected to the master apparatus 51 via the optical cable 170-k. The optical cable 170-k may be a connector-equipped cable comprising connectors at both ends.

In Fig. 12, four optical cables 170-1, 170-2, 170-3, and 170-4 are shown, but a multicore optical cable may be used instead of the optical cable 170-k. The master apparatus 51 is an optical communication apparatus disposed at a location away from the patient table 130 and functions as an optical controller for optical communication. The master apparatus 51 is connected to the control unit 142 and the image reconstruction unit 146 in the same manner as the optical wireless unit 50. The master apparatus 51 may have the same optical wireless communication function as that of the optical wireless unit 50.

With the optical wireless unit 11, wired communication using the optical cable 170-k can be performed instead of the optical wireless communication or in combination with the optical wireless communication. The optical wireless unit 11 may have a configuration in which a communication method is selected based on a control signal from the optical wireless unit 50 or the master apparatus 51. Additionally, the optical wireless unit 11 may have a configuration in which a communication method is selected in conjunction with the presence or absence of the connection of the optical cable 170-k. A configuration may be employed in which at least one of the optical wireless unit 10-1 or 10-2 shown in Fig. 11 comprises an optical cable connector, similar to the optical wireless unit 11.

Effects of Embodiment

With the patient table 130 comprising the optical wireless unit 10 or 11 according to the embodiment and the MRI apparatus 100 including the patient table 130, the following effects can be obtained.

(1) Since the optical wireless unit 10 is disposed at the end portion of the leg portion 130B on the side opposite to the gantry 110 or the side surface portion of the leg portion 130B, which is outside the movement range of the top plate 130A, the optical wireless unit 10 is exposed regardless of the position of the top plate 130A, and continuous and stable communication with the optical wireless unit 50 disposed on the ceiling or the like of the MRI room 310 is possible at all times.

(2) Since the A/D conversion unit 134-k is disposed inside the top plate 130A to correspond to the coil port 132-k of the top plate 130A, the digitization and the E/O conversion of the signal can be performed near the coil port 132-2, thereby suppressing noise interference and signal loss.

(3) For the configurations of the receive coil units 200, 204, 205, and 206, the signal transmission cables 220, 224, 225, and 226, and the coil port 132-k, existing configurations can be used.

(4) Since the data is transmitted through optical wireless modulation by the optical wireless unit 10 disposed on the leg portion 130B, and the data is received by the optical wireless unit 50 and transmitted to the image reconstruction unit 146, this configuration allows for data transmission while avoiding electromagnetic interference. In addition, optical cable wiring is not required between the patient table 130 and the image reconstruction unit 146.

(5) Since the optical cable connection is not required between the patient table 130 and the image reconstruction unit 146, the patient table 130 can be easily configured as a movable patient table (dockable patient table).

(6) By connecting the A/D conversion unit 134-k corresponding to each of the plurality of coil ports 132-k provided on the top plate 130A to the optical wireless unit 10 (or the optical wireless unit 11) via the optical cable 136-k, the signal obtained from the activated receive coil unit can be selectively transmitted through optical wireless communication.

(7) With the MRI apparatus according to the present embodiment, the influence of noise components can be suppressed, thereby obtaining a high-quality image.

Others

The present invention is not limited to the above-mentioned embodiment, and various modifications are possible within the scope of the technical concept of the present disclosure without departing from its scope as defined by the claims.

Explanation of References

10, 10-1, 10-2: optical wireless unit
11: optical wireless unit
13-1, 13-2, 13-3, 13-4: optical cable connector
40-1, 40-2: O/E converter
42-1, 42-2: E/O converter
44: P/S converter
46: optical wireless transmission unit
47: optical wireless reception unit
48: S/P converter
50, 50-1, 50-2: optical wireless unit
51: master apparatus
100: MRI apparatus
102: subject
104: static magnetic field generating magnet
106: gradient magnetic field coil
108: RF transmit coil
110: gantry
112: high-frequency magnetic field generator
114: receiver
116: gradient magnetic field power supply
118: sequencer
120: bore
130: patient table
130A: top plate
130B: leg portion
132, 132-1, 132-2, 132-3, 132-4: coil port
134, 134-1, 134-2, 134-3, 134-4: A/D conversion unit
136-1, 136-2, 136-3, 136-4: optical cable
140: controller
142: control unit

146: image reconstruction unit
150: operation unit
152: console
160: A/D converter
162: P/S converter
164: E/O converter
166: O/E converter
168: S/P converter
170-1, 170-2, 170-3, 170-4: optical cable
200: receive coil unit
200-1, 200-2, 200-3, 200-4, 200-j: coil element
202: decoupling power supply
210-1, 210-2, 210-3, 210-4, 210-j: decoupling circuit
220, 224, 225, 226: signal transmission cable
310: MRI room
312: machine room

**Claims**

1. A patient table comprising:

   a top plate (130A) that moves with a subject (102) placed thereon;
   a leg portion (130B) that supports the top plate;
   a signal conversion unit (134) that is disposed inside the top plate and that is connected to a receive coil unit (200) which receives a signal generated by the subject, the signal conversion unit including an A/D converter (160) that converts the signal obtained from the receive coil unit into a digital signal, and an electrical-to-optical converter (164) that converts the digital signal into an optical signal;
   an optical cable (136) that is disposed inside the top plate and the leg portion and that transmits the optical signal output from the signal conversion unit; and
   an optical wireless unit (46) that is disposed on the leg portion at a position outside a movement range of the top plate, that is connected to the optical cable, and that transmits the optical signal via optical wireless communication.

2. The patient table according to claim 1,
   wherein the optical wireless unit (46) is disposed at an end portion of the leg portion in a direction in which the top plate moves.

3. The patient table according to claim 1 or 2,
   wherein the optical wireless unit (46) is disposed on a side surface portion of the leg portion.

4. The patient table according to any one of claims 1 to 3,

   wherein the top plate (130A) includes a coil port (132) including a connector to which a signal transmission cable of the receive coil unit is connected, and
the signal conversion unit is disposed to correspond to the coil port.

5. The patient table according to claim 4,

   wherein the top plate includes a plurality of the coil ports, and
   the signal conversion unit is provided for each of the plurality of coil ports.

6. The patient table according to any one of claims 1 to 5,
   wherein a plurality of the signal conversion units and a plurality of the optical cables are provided.

7. The patient table according to claim 6,
   wherein the optical wireless unit includes a switch for selecting a signal to be activated from the plurality of signal conversion units.

8. The patient table according to claim 6 or 7,
   wherein the optical wireless unit includes a circuit that serializes signals obtained from a plurality of the receive coil units connected to the plurality of signal conversion units.

9. The patient table according to any one of claims 1 to 8,
   wherein the signal conversion unit includes a digital processing circuit.

10. The patient table according to claim 9,
    wherein the digital processing circuit includes one or both of a decimator that thins out digitized data and a circuit that performs encoding.

11. The patient table according to any one of claims 1 to 10,
    wherein a plurality of the optical wireless units are provided.

12. The patient table according to any one of claims 1 to 11,
    wherein the optical wireless unit includes an optical cable connector (13-k) for wired connection to an optical communication apparatus disposed at a location away from the patient table.

13. A magnetic resonance imaging apparatus comprising:

    the patient table (130) according to any one of claims 1 to 12;
    a gantry (110) that generates a static magnetic field, a gradient magnetic field, and a high-frequency magnetic field in an imaging space;

a second optical wireless unit that performs optical wireless communication with a first optical wireless unit which is the optical wireless unit; and
an image reconstruction unit (146) that reconstructs an image based on data of a nuclear magnetic resonance signal acquired via the second optical wireless unit.

14. The magnetic resonance imaging apparatus according to claim 13,

wherein the second optical wireless unit is disposed on a ceiling or a wall of a room where the gantry is disposed, and
preferably, the second optical wireless unit communicates with the first optical wireless unit via visible light communication and also serves as a light for the room.

15. The magnetic resonance imaging apparatus according to claim 13 or 14, wherein a plurality of the second optical wireless units are provided.

## FIG. 1

FIG. 2

MRI APPARATUS 100

STATIC MAGNETIC FIELD GENERATING MAGNET 104

108 102 200 106

130

HIGH-FREQUENCY MAGNETIC FIELD GENERATOR 112

RECEIVER 114

GRADIENT MAGNETIC FIELD POWER SUPPLY 116

SEQUENCER 118

CONTROLLER 140

OPERATION UNIT 150

FIG. 3

FIG. 4

# FIG. 5

FIG. 6

EP 4 660 654 A1

# FIG. 7

BIDIRECTIONAL OPTICAL
WIRELESS COMMUNICATION

# FIG. 8

GANTRY ←

132-1  130A  220  132-2  10

132-4  102  200  132-3  130B

10

EP 4 660 654 A1

# FIG. 9

## FIG. 10

FIG. 11

EP 4 660 654 A1

FIG. 12

EP 4 660 654 A1

**EUROPEAN SEARCH REPORT**

Application Number

EP 25 18 1167

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2022/265496 A1 (XUE TING QIANG [CN] ET AL) 25 August 2022 (2022-08-25) | 1-4,9, 10,13,14 | INV. G01R33/30 |
| Y | * paragraphs [0033], [0036], [0054] – [0060]; figures 10, 11 * | 5-8,11, 15 | A61B5/00 G01R33/36 |
| A | | 12 | |
| | ----- | | |
| Y,D | JP 2007 144192 A (GEN ELECTRIC) 14 June 2007 (2007-06-14) * paragraphs [0007] - [0010], [0024]; figures 1, 2 * * paragraph [0016]; figure 5 * | 5-8,11, 15 | |
| | ----- | | |
| A,D | JP 2014 061282 A (TOSHIBA CORP; TOSHIBA MEDICAL SYS CORP) 10 April 2014 (2014-04-10) * paragraphs [0026], [0030], [0039], [0052], [0058] - [0064] * | 7-10 | |
| | ----- | | |

| | **TECHNICAL FIELDS SEARCHED (IPC)** |
|---|---|
| | G01R A61B |

The present search report has been drawn up for all claims

| Place of search | Date of completion of the search | Examiner |
|---|---|---|
| Munich | 21 October 2025 | Raguin, Guy |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P4C01)

## ANNEX TO THE EUROPEAN SEARCH REPORT
## ON EUROPEAN PATENT APPLICATION NO.

EP 25 18 1167

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2025

| Patent document cited in search report | Publication date | Patent family member(s) | | Publication date |
|---|---|---|---|---|
| US 2022265496 A1 | 25-08-2022 | CN | 215348948 U | 31-12-2021 |
| | | US | 2022265496 A1 | 25-08-2022 |
| JP 2007144192 A | 14-06-2007 | DE | 102006056453 A1 | 31-05-2007 |
| | | JP | 2007144192 A | 14-06-2007 |
| | | NL | 1032934 C2 | 02-11-2007 |
| | | US | 7173426 B1 | 06-02-2007 |
| JP 2014061282 A | 10-04-2014 | JP | 6430107 B2 | 28-11-2018 |
| | | JP | 2014061282 A | 10-04-2014 |
| | | US | 2015168513 A1 | 18-06-2015 |
| | | WO | 2014034817 A1 | 06-03-2014 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2014061282 A
- JP 2014230610 A

- JP 2007144192 A